# EUROPEAN PATENT APPLICATION

(11) **EP 1 428 526 A1**
(43) Date of publication of application: **16.06.2004**
(21) Application number: 02080260.9
(22) Date of filing: 13.12.2002
(51) Int. Cl.: A61K 9/19, A61K 9/20, A61K 31/4422, A61K 31/5513

(54) **Formulation for fast dissolution of lipophilic compounds**

(71) Applicant: Rijksuniversiteit Groningen, 9712 CP Groningen (NL)
(72) Inventor: The designation of the inventor has not yet been filed
(74) Representative: Prins, Adrianus Willem, Mr. Ir.

(57) **Abstract**

The present invention relates to a pharmaceutical composition comprising at least one lipophilic compound and at least one glass of a sugar, a sugar alcohol, a mixture of sugars, a mixture of sugar alcohols or a mixture of at least one sugar and at least one sugar alcohol, wherein the lipophilic compound is incorporated in the sugar glass. The present invention further relates to a method for manufacturing such a pharmaceutical composition.

## Description

The present invention relates to a pharmaceutical formulation for fast release of lipophilic compounds.

Many lipophilic drugs exhibit a low and irregular bioavailability when they are administered orally. This is caused by their poor water solubility, which results in slow dissolution in the aqueous gut lumen. Because of their manufacturing difficulties and stability problems, only few formulations that tackle this problem have been brought to the market. However, the need for formulations containing lipophilic compounds has increased drastically during the last decade, mainly due to the scanning methods for finding new chemical entities with biological activity. Therefore, one of the most active areas in pharmaceutical engineering is the development of a formulation with a high and reproducible absorption into the human or animal body.

It has been suggested to use solid dispersions of lipophilic compounds. Solid dispersions typically consist of a water-soluble matrix that incorporates very small amorphous particles or even separate molecules of the lipophilic compound.

A technique to make solid dispersions is the fusion technique wherein both the matrix material (carrier) and drug are heated. The drug is dissolved in or melted together with the matrix material. Because miscibility of drug and matrix material is often problematic, the mixture often tends to phase separate.( Greenhalgh, D.J.; Williams, A.C.; Timmins, P.; York, P., J. of Pharmaceutical Sciences, 1999, 88, 1182-1190). E.g. during cooling the melt bears the risk of phase separation to yield two liquid phases or crystals of drug, carrier or both. Surfactants like Gelucire® can be used to overcome this problem (Damian, F.; Blaton, N.;Kinget, R.; Van den Mooter, G., International J. of Pharmaceutics, 2002,, 244, 87-98). Furthermore, fusion techniques only apply to heat stable materials, for example for sugar matrices heat-stability at temperatures of about 170 °C or higher has been found to be required.

Another technique to produce solid dispersions is the solvent technique. The solvent technique requires dissolution of both drug and carrier in one solution followed by drying, e.g. spray drying, vacuum drying, super critical drying or freeze drying. Several strategies can be distinguished. The use of extremely low drug concentrations is a way to dissolve both carrier and drug in water, but requires the evaporation of tremendous amounts of solvent, making the process uneconomic and impractical. Solubilisers like cyclodextrins or surfactants may be used to increase the aqueous solubility of the drug significantly, however a high amount of solubilisers is generally required. This results in solid dispersions that mainly consist of solubilisers and hence largely affect the physical properties of the matrix. Besides solubilisers are not always tolerated well in the body or may even be toxic.

Another strategy is to use one solvent in which both drug and carrier are dissolved. Chloroform Betageri, G.V.; Makarla, K.R., International J. of Pharmaceutics, 1995, ***126*,** 155-160 or dichloromethane (Damian et al. 2002, vide supra) are frequently used to dissolve both drug and matrix (in particular poly(vinyl-pyrrolidone) (PVP)) simultaneously. These solvents are impractical for freeze drying and commonly used in vacuum drying, preferably at elevated temperatures. With this drying method there is an increased risk for phase separation. Furthermore, these solvents only dissolve matrix materials of a relatively low polarity very well, which limits the number of matrix materials that can be used with these solvents. Besides, due to their toxicity, problems can be expected with residual solvents still present after drying.

Another strategy is the use of supercritical solvent, which involves a complicated procedure.

Another strategy for the dissolution of both drug and carrier is the use of a co-solvent. Water and ethanol, or methylene chloride and ethanol are examples encountered in literature. However, because of the low melting temperature of ethanol, mixtures with ethanol are in practice not considered to be suitable for normal freeze drying. Again, other drying methods bear the risk of phase separation in the co-solvent mixture, because the two solvents will in general evaporate not equally fast.

It is an object of the present invention to provide a pharmaceutical composition, in particular a solid composition, comprising a lipophilic compound, which is adequatily solubilised in an aqueous solution, preferably in such a way that this compound is rapidly dissolved in an aqueous solution, in particular in such a way that it is rapidly dissolved in vivo. It is in particular an object to provide such a formulation comprising a crystallisable lipophilic compound.

Accordingly, the present invention relates to a pharmaceutical composition comprising at least one lipophilic compound and at least one glass of a sugar, a sugar alcohol, a mixture of sugars, a mixture of sugar alcohols or a mixture of at least one sugar and at least one sugar alcohol, wherein the lipophilic compound is incorporated in the sugar glass.

Thus it has been found possible to provide a formulation with a desirable release pattern, resulting in a very good absorption after application to a human or animal.

A lipophilic compound in a composition according to the invention has further been found to have a good stability against degradation, e.g. by oxidation, and/or isomerisation.

A lipophilic compound as used herein is defined as a hydrophobic compound - more in particular a hydrophobic organic compound - having a solubility in an aqueous environment at 37 °C of less than 33 g/L. In particular, the term lipophilic compound is used to describe a compound that has a solubility of less than 33 g/L under the conditions, in particular the pH, at the site in vivo (e.g. in the stomach, in the intestines, subcutaneous) where the compound is intended to become available to the body (in particular where the compound is dissolved to be absorbed by the body). Thus, for example a lipophilic compound intended to dissolve in the stomach, has a solubility below 33 g/l in gastric fluid (pH of about 1-3) and a lipophilic compound to be dissolved in the intestines has a solubility below 33 g/l in intestinal fluid (typically up to about pH 7.4).

The incorporation is preferably such that the lipophilic compound is mono-molecularly distributed over the matrix or dispersed in nano- or microparticles. In case of mono-molecular distribution the lipohilic compound is typically present in an amorphous state. A micro- or nano-particle of the lipophilic compound may be solid, such as crystalline or amorphous.

The term sugar as used hereafter is intended to include oligo-sugars, poly-sugars and sugar alcohols (also known as polyols). The sugar glass serves as a matrix for the lipophilic compound or compounds. In principle, the sugar glass can comprise any amorphous pharmaceutically acceptable sugar and/or derivative thereof. Suitable are for example oligo- and polysaccharides, including sugar alcohols thereof. Examples of such oligo- and polysaccharides are oligo- and polysaccharides based upon glucose units (e.g. dextran, maltodextrin) fructose units (e.g. inulin, levan), mannose units (e.g. mannan) and oligo- and polysaccharides based upon galactose units (e.g. galactan). Particularly suitable are non-reducing sugars. Preferred are fructans, such as those mainly containing β-1, 2 bonds as in inulin or mainly containing β-2,6 bonds as in levan. Suitable fructans are e.g. those referred to in WO 00/78817.

In principle any processable sugar material can be used as a matrix. It has however been found by the inventors that it is favourable when the sugar has a relatively low dissolution rate in water, in comparison to trehalose and to sucrose. It has been found that when compositions wherein the matrix (mainly) consists of relatively fast dissolving sugars, at least a partial crystallisation of the lipophilic compound occurs. After this lipophilic compound is crystallised, dissolution of the crystals has been found to be very slow. It has also been found that this in turn may be detrimental to the effective uptake in the body.

Accordingly, the inventors found that the lipophilic compound in a pharmaceutical composition according to the invention is solubilised more effectively when the sugar dissolves relatively slow. Furthermore, thus the absorption of the lipophilic compound in the body can be improved.

As a result, it has been found possible to improve the dissolution rate of a lipophilic compound by selecting a matrix material based upon the solubility of the lipophilic compound. In particular, it has been found that the effective dissolution rate of a lipophilic compound is very well in an aqueous medium, in case the dissolving time of a matrix material is at least 2 min, preferably at least 5 min. Very good results have been achieved with a matrix material that has a dissolving time of 30 min of less. The dissolving time for the matrix material as used herein is the time as determined by a standard dissolution test on a Prolabo dissolution apparatus (as indicated in Example 1: in 1000 ml water at 37 °C, 100 rpm, with a tablet with a 9 mm diameter and 1.3-1.5 mm thickness, porosity of 20-25%).

Further it has been found advantageous to provide a composition with a matrix that exhibits a high viscosity both inside the tablet and/or in the boundary layer around the tablet during dissolving. Preferably, the dynamic viscosity in the boundary layer of the tablet during dissolution (at 37 °C) is at least 60 mPa·s, as measured by Brookfield rotation viscosimetry.

Very good results with respect to the effective solubilisation of the lipophilic compound, such that its absorption is well, have been achieved with a composition wherein the lipophilic compound is incorporated in a glass of a poly-saccharide. A polysascharide has been found particularly suitable to serve as the sugar glass in a fast release formulation. Very good results have been obtained with a fructan, in particular an inulin, as the polysaccharide.

The number average degree of polymerisation (DP)of the sugar molecules of the sugar glass is preferably at least 5, more preferably at least 6, even more preferably at least 10. The DP is readily determinable by HPLC.

In practice, the DP will generally be less than 1000. Good results have been achieved with a composition wherein the sugar molecules of the sugar glass have a DP of 60 or less, preferably 30 or less, more preferably 25 or less. Very good results have been achieved with a composition wherein the sugar molecules of the sugar glass have a DP of 11-24.

With respect to stability it has been found advantageous to choose a sugar having a glass transition temperature of at least 40 °C, more in particular a sugar having a glass transition temperature of at least 40 °C after being saturated with moisture (after exposure to a humid environment). The upper limit for the glass temperature is not particularly critical and depends inter alia on the type of sugar and the DP. For examples sugar glasses such as fructans typically have a Tg of up to about 170 °C, although it is within the scope of the invention to that the sugar-glass has a higher Tg, e.g. of 200 °C or even higher.

A lipophilic compound in a composition according to the invention can be a natural or a synthetic compound. The present invention has been found particularly suitable for providing a pharmaceutical composition comprising a synthetic lipophilic compound.

The present invention has been found particular suitable for providing a pharmaceutical composition comprising a crystallisable lipophilic compound, in particular a compound that is capable of forming crystals when precipitating from a polar solution such as an aqueous solution, using a conventional technique. Of known pharmaceutical compositions comprising a crystallisable lipophilic compound, it has been found that the dissolution rate is negatively affected due to the formation a crystallised lipophilic compound phase during dissolution of the composition.

Accordingly, in a preferred aspect of the present invention is distinguished from a pharmaceutical compound only comprising one or more non-crystallisable lipophilic compounds - such as cannabinoids, in particular such as Δ⁹-tetrahydrocannabinol (THC) - as the lipophilic compound(s) incorporated in the sugar glass. (THC has been reported to be non-crystalisable in Goani, Y.; Mechoulam, R., J. of the American Chemical Society, 1971, ***93***, 217-224).

A lipophilic compound in a composition according to the invention is preferably a biologically active compound. Suitable examples thereof are antipsychotic drugs, preferably haloperidol, triflapromazine, flufenazine, olanzapine, clozapine or a functional derivative thereof; benzodiazepines, preferably lorazepam, flurazepam, triazolam, clonazepam, chlordiazepoxide, temazepam, oxazepam, clorazepate, diazepam, alprazolam, prazepam, loprazolam, lormetazepam, nitrazepam, triazolam or a functional derivative thereof; calcium antagonists, preferably barnidipine, nifedipine or a functional derivative thereof; corticosteroids, preferably methyl prednisolon, triamcinolon, budesonide, flixotide and dexamethason or a functional derivative thereof; diaretica, preferably flurosemide, spironolacton or a functional derivative thereof; heart glycosides, preferably digoxine, digitoxine or a functional derivative thereof; hormones, preferably progesterone, testosteron or a functional derivative thereof; lipophilic alkaloids, preferably codein or a functional derivative thereof; lipophilic antibiotics, preferably eryrthromycin lauryl sulfate, metronidazole, rifampin, minocycline, doxycycline, or a functional derivative thereof; lipophilic polypeptides, preferably cyclosporine or a functional derivative thereof; non steroidal antiinflammatory drugs, preferably diclofenac, piroxican, indomethacin, paracetamol or a functional derivative thereof, vitamins, preferably vitamin D, vitamin A, vitamin E or vitamin K, griseofulvin; lidocaine; ondansetron.HCl; methyl butazone and theophilline.

Very good results have been achieved with benzodiazepines in particular with diazepam and with calcium antagonists, in particular with nifedipine.

The amount of lipohilic compound(s) in a composition according to the present invention is preferably up to 63 wt. % based upon the weight of sugar glass and lipophilic compound(s), more preferably up to 50 wt. %. For practical reasons the amount of lipophilic compound(s) is preferably at least 0.0001 wt. %, more preferably at least 0.001 wt %.

Besides sugar glass and lipophilic compound a composition according to the invention may further comprise one or more additives such as flavouring agents, colourants, binders, fillers, filler-binders, lubricants, desintegration aids and/or other pharmaceutically acceptable additives. The total amount of additives is for practical reasons preferably less than 99.95 wt. % based upon the total weight of the composition. In particular the use of desintegration aids, fillers, filler-binders and/or binders in a formulation according to the invention may be advantageous.

Very good results have been achieved with a composition according to the invention essentially consisting of the sugar glass and lipophilic compound(s).

A pharmaceutical composition according to the present invention may be processed into any form, in particular into any solid form.

Preferably a composition according to the invention is a tablet such as a normal oral tablet, a sublingual tablet, a buccal tablet or an orally disintegrating or dissolving tablet, a capsule, a lozenge, an enema, a suppository, a product for transdermal administration, a powder for pulmonary administration, or a rod or suspension for subcutaneous or intramuscular administration.

A composition according to the invention, can in principle be made in any way. Typically, the sugar and lipophilic compound are dissolved in a solvent (simultaneously or by first dissolving the separate compounds and thereafter mixing the solutions), and then dried.

In principle it is possible to dissolve sugar and lipophilic compound in the same solvent, for example water.

A preferred technique for preparation of a composition according to the invention is the so-called co-solvent technique, wherein sugar and lipophilic compound are first dissolved in separate solvents, which solvents are then mixed, whereafter the mixture is dried, e.g. by freeze-drying, spray drying, vacuum drying or super critical drying. An advantage of the co-solvent technique, is the possibility to prepare a preparation with a high load of lipophilic compound in comparison to a single solvent technique, such as a technique wherein water is used as the solvent.

The solvent for the sugar (matrix-solvent) can be any solvent in which the sugar dissolves to a sufficient degree and is miscible with the co-solvent. The solvent preferably has a high vapour pressure and in case of freeze-drying as the drying technique preferably a high melting point. Examples of possible matrix-solvents are dimethylsulfoxide and water. Water is particularly preferred, inter alia since it exhibits a high vapour pressure and allows a fast drying time.

The lipophilic compound is preferably dissolved in a different solvent (the co-solvent) than the solvent used for the sugar. A suitable co-solvent should therefore be capable of dissolving the drug of interest, it should preferably be miscible with the matrix-solvent (such as water) and preferably exhibit a high vapour pressure. In case the drying technique is freeze-drying it is further practical that the co-solvent has a high melting point. A skilled person will know how to choose suitable solvents. Particular suitable co-solvents are for example co-solvents selected from the group consisting of dimethylsulfoxide, N,N-dimethylformamide, acetonitrile, ethyl acetates and C1-C6 alcohols. Highly preferred are C2-C4 alcohols, e.g. ethanol, n-propyl alcohol and tertiary butyl alcohol, of which tertiary butyl alcohol (TBA) is particularly preferred.

The sugar in the solvent for the matrix and the lipophilic compound in co-solvent are then mixed, after which the solvents are evaporated to obtain a sugar glass with the lipophilic compound incorporated therein. The ratios between the lipophilic compound, the solvent, water and the sugar should be chosen in such a way that a sufficiently stable solution is obtained, the skilled person will know how to choose such conditions on the basis of common general knowledge. Optionally a surfactant (e.g. Tween® 80 and/or solubiliser (e.g. a cyclodextrin or gelucire®) may be added, but highly satisfactory results have been obtained in the absence of a surfactant and a solubiliser. A solution is considered to be sufficiently stable if no clouding is visible in the solution within the time of processing, e.g. within 10 min, in case of freeze-drying. In a freeze-drying process the solution is preferably clear until frozen. The maximum water content after drying is preferably less than 3 wt %.

Freeze drying is highly preferred, inter alia because it has been found to be suitable for preparing a composition with a very good stability, in combination with a desirable release of lipophilic compound(s). Preferably the freeze drying is carried out below the Tg' of the freeze concentrated fraction (as indicated in D.L. Teagarden, Eur. J. Pharm Sci, 15, 115-133, 2002). Thus it has been found possible to form a porous cake of the pharmaceutical composition, that can be processed very suitably into a end-product.

Additives (other than the surfactant/solubiliser), which may optionally be present in a pharmaceutical composition according to the invention are preferably added to the composition after the composition has been dried.

The present invention further relates to the use of a sugar glass in the manufacture of a medicament in solid form, comprising as active agent a lipophilic compound for increasing the bioavailability or dissolution rate of the active agent. Preferred sugar glasses and preferred lipophilic compounds are those described herein.

The invention is now further illustrated by the following example.

### Example 1.

Dissolution of sugar glass dispersions with sucrose, trehalose, and two inulins with degrees of polymerization (DP) of 11 respectively 24 containing diazepam or nifedipine as lipophilic compounds.

### Materials

The following materials were used as supplied: tertiary butanol (TBA), Diazepam, Nifedipine, trehalose, sucrose, Poly-(oxyethylene sorbitan) Tween®80 and Anthrone reagent. Two Inulins, type TEX 803! and HD001111, having a degree of polymerization (DP) of 24 and 11, respectively, were provided by Sensus, Roosendaal, The Netherlands. The water used was demineralised in all cases.

### Methods

### Production of the solid dispersions

To produce a solid dispersion with 10%w/w drug, 1.20 mL of a 150mg/mL sugar in water solution was added to 0.80 mL of a 25 mg/mL drug solution in TBA and mixed in a 20 mL vial. This results in a concentration of 90mg/mL of sugar and 10mg/mL of drug in a solvent that consists of 40%v/v TBA. The vial was shaken manually before the solution was immersed in liquid nitrogen for a few minutes. After that, the vials were freeze dried using a Christ model Alpha 2-4 lyophilizer, Salm and Kipp, Breukelen, The Netherlands. The frozen solutions were lyophilized (condensor temperature - 53°C) at a shelf temperature of -35°C and a pressure of 0.220 mbar for 1 day. Subsequently, the shelf temperature was gradually raised to 20°C while the pressure was decreased to 0.05 mbar for another day. After removing the samples from the freeze drier, they were placed in a vacuum desiccator at room temperature for 1 day.

### Preparation of physical mixtures (lipophilic compound not incorporated in sugar glass)

Physical mixtures were prepared of both diazepam and nifedipine with sucrose, trehalose, inulin (DP11) or inulin (DP24). All sugars were freeze dried from TBA/water mixtures of 40%v/v TBA, without any drug. Drug and sugar were gently mixed with a spoon in a mortar to yield a physical mixture of 10%w/w drug. After that the mixtures were stored in a desiccator over silicagel.

### Tabletting

Both the solid dispersions as well as the physical mixtures containing nifedipine or diazepam were compressed to tablets (thickness of 1.3-1.5 mm, porosity of 20-25 %) of 200mg (13 mm diameter) or 100mg (9 mm diameter) respectively on a ESH compaction apparatus, ESH testing limited. The maximum force of 5 kN was reached in 1 second. After compaction the tablets were weighed and subsequently submitted to dissolution experiments.

### Dissolution experiments

Dissolution was performed on a Prolabo dissolution apparatus, Rowa Techniek B.V.. All experiments were conducted at 37°C in 1000 mL under constant stirring with 100 rpm. Tablets prepared from physical mixtures and solid dispersions were analysed in triplicate. Diazepam tablets were dissolved in water. Nifedipine tablets were dissolved in 1%w/v Tween80 in water to ensure sink conditions.

### Analysis of concentrations in dissolution media

Diazepam and Nifedipine were analysed spectrophotometrically at 230 nm and 340 nm respectively. To determine the sugar concentrations the Anthrone assay was used. (Scott et al. 1953) Samples were taken at appropriate intervals during the dissolution. During diazepam experiments 1.00mL was taken from the dissolution medium mixed with 2.00 mL Anthrone reagent 0.1%w/v in sulfuric acid. Due to the enthalpy of mixing, the sample was heated to its boiling point. The boiling mixture was then cooled to room temperature. After 45 minutes the sample was vortexed and 200 mL of sample was analysed in the plate reader at 630 nm. Nifedipine samples of 0.25 mL were diluted with 0.75mL water before they were submitted to the Anthrone assay. In every Anthrone assay a calibration curve of the appropriate sugar in the appropriate medium was established.

### Results

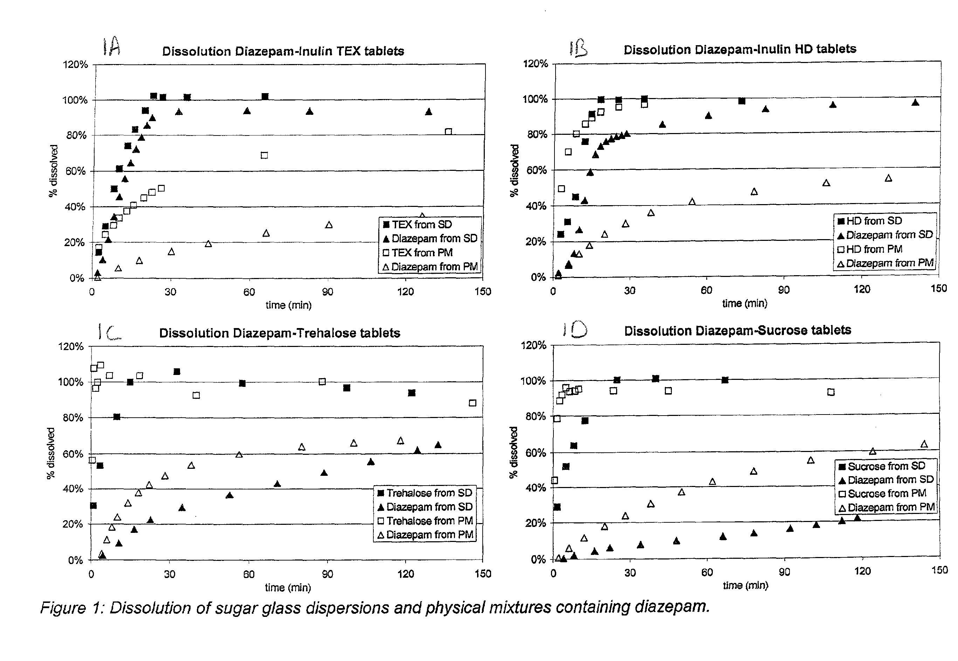

The dissolution of diazepam from sugar glass dispersions with the four different sugars is depicted in Figure 1A-D. In the legends to the figures: "TEX" refers to inulin with a DP of 24, "HD" to inulin with a DP of 11, "SD" to the solid dispersion and "PM" to the physical mixture.

Both inulins show fast release of diazepam from the solid dispersions. The inulins dissolve in the same rate as the diazepam. For the physical mixtures of the inulins with diazepam, the diazepam is dissolved slower. The inulin DP24 dissolution seems to be accelerated in case of a solid dispersion.

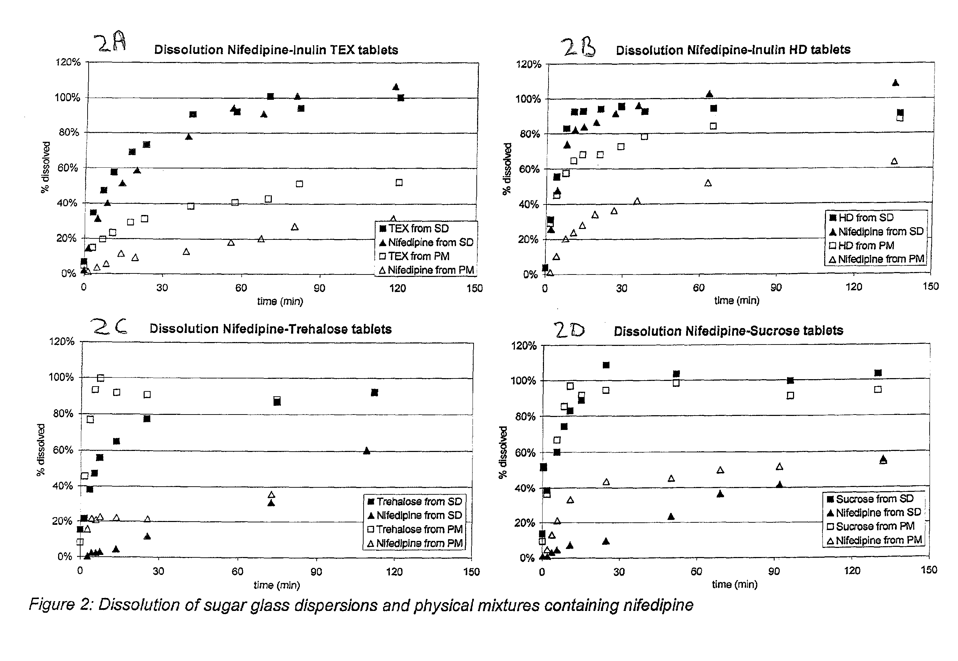

A complete different behaviour can be observed with both sucrose and trehalose. The release of diazepam from a physical mixture is fast compared to the solid dispersions. It is clear from these graphs that both sucrose and trehalose dissolve faster compared to the inulins especially for physical mixtures. As can be seen form Figure 2A-D, similar results were obtained with nifedipine.

In all dissolution experiments with solid dispersions containing sucrose or trehalose, crystallisation was observed visually. After about thirty minutes when the sugar was completely dissolved, a porous structure was still visible in the dissolution beaker. This porous structure was gently taken out of the beaker and calorimetrically analysed. It turned out that the material consisted of pure crystalline drug.

## Claims

1. A pharmaceutical composition comprising at least one lipophilic compound and at least one glass of a sugar, a sugar alcohol, a mixture of sugars, a mixture of sugar alcohols or a mixture of at least one sugar and at least one sugar alcohol, wherein the lipophilic compound is incorporated in the sugar glass.

2. A pharmaceutical composition according to claim 1, wherein said sugar or sugar alcohol is an oligosaccharide, an oligosaccharide alcohol, a polysaccharide or a polysaccharide alcohol.

3. A pharmaceutical composition according to claim 2, wherein the sugar is a fructan.

4. A pharmaceutical composition according to claim 3, wherein the fructan is an inulin.

5. A pharmaceutical composition according to any one of the claims 2-4, wherein the number average degree of polymerisation is at least 5, preferably 6-30, more preferably 10-25.

6. A pharmaceutical composition according to any one of the preceding claims, comprising a crystallisable lipophilic compound.

7. A pharmaceutical composition according to any one of the preceding claims, comprising a lipophilic compound having a solubility in an aqueous environment at 37 °C of less than 33 g/L.

8. A pharmaceutical composition according to any one of the preceding claims, comprising at least one lipophilic compound, selected from the group consisting of antipsychotic drugs (such as haloperidol, triflapromazine, flufenazine, olanzapine and clozapine), benzodiazepines (such as lorazepam, flurazepam, triazolam, clonazepam, chlordiazepoxide, temazepam, oxazepam, clorazepate, diazepam, alprazolam, prazepam, loprazolam, lormetazepam, nitrazepam and triazolam) calcium antagonists (such as barnidipine and nifedipine), corticosteroids (such as methyl prednisolon, triamcinolon, budesonide, flixotide and dexamethason), diaretica (such as flurosemide and spironolacton), heart glycosides (such as digoxine and digitoxine), hormones (such as progesterone and testosteron), lipophilic alkaloids (such as codein), lipophilic antibiotics (such as eryrthromycin lauryl sulfate, metronidazole, rifampin, minocycline and doxycycline), lipophilic polypeptides (such as cyclosporine), non steroidal anti inflamatory drugs (such as diclofenac, piroxican, indomethacin and paracetamol), vitamins (such as vitamin D, vitamin A, vitamin E and vitamin K), griseofulvin, lidocaine, ondansetron.HCl, methyl butazone and theophilline.

9. A pharmaceutical composition according to any one of the preceding claims, in the form of a tablet (such as a normal oral tablet, a sublingual tablet, a buccal tablet or an orally desintegrating or dissolving tablet), a capsule, a lozenge, an enema, a suppository, a product for transdermal administration, a powder for pulmonary administration, or a rod or suspension for subcutaneous or intramuscular administration.

10. A pharmaceutical composition according to any of the preceding claims, comprising more than 37 wt. % and less than 100 wt. % sugar glass, based upon the total weight of sugar glass and lipophilic compounds.

11. A method for preparing a pharmaceutical composition according to any of the preceding claims, wherein a solution of the sugar, sugar alcohol, mixture of sugars, mixture of sugar alcohols or mixture of at least one sugar and at least one sugar alcohol and the lipophilic compound is dried, preferably by freeze drying, to form the sugar glass wherein the lipophilic compound is incorporated.

12. A method for preparing a pharmaceutical composition according to claim 11, wherein the sugar, sugar alcohol, mixture of sugars, mixture of sugar alcohols or mixture of at least one sugar and at least one sugar alcohol is dissolved in a first solvent, preferably water, and said lipophilic compound is dissolved in a co-solvent, preferably a C1-C6 alcohol, next the first solvent and the co-solvent are mixed, and thereafter the resultant mixture is dried.

13. Use of a sugar glass in the manufacture of a medicament in solid form, comprising as active agent a lipophilic compound for increasing the bioavailability or dissolution rate of the active agent.
